# EUROPEAN PATENT APPLICATION

(11) **EP 2 752 147 A1**
(43) Date of publication of application: **09.07.2014**
(21) Application number: 12828429.6
(22) Date of filing: 16.05.2012
(51) Int. Cl.: A61B 1/00

(54) **CAPSULE-TYPE ENDOSCOPE**

(30) Priority: 31.08.2011 JP 2011189701
(71) Applicant: Olympus Corporation, Shibuya-ku Tokyo 151-0072 (JP)
(72) Inventor: IGARASHI, Takatoshi, Tokyo 151-0072 (JP); MAKINO, Yukiharu, Shibuya-ku, Tokyo 151-0072 (JP); FUJIMORI, Noriyuki, Shibuya-ku, Tokyo 151-0072 (JP)
(74) Representative: Winter, Brandl, Fürniss, Hübner, Röss, Kaiser, Polte - Partnerschaft
(86) International application number: PCT/JP2012/062468
(87) International publication number: WO 2013/031300

(57) **Abstract**

In a capsule endoscope 10, inside a housing 11, a plurality of connecting sections 27 of a circuit board 20 formed by arranging a plurality of board sections 21 to 26 to one another in a row via the connecting sections 27 are bent at 180 degrees and the plurality of board sections 21 to 26 are arranged such that principal planes of the respective board sections 21 to 26 are orthogonal to a center axis O of the housing 11. The capsule endoscope 10 includes a transducer section 22C, a first image pickup chip 22A connected to the transducer section 22C via two signal lines and configured to generate a clock signal and acquire first image data according to the generated clock signal, a second image pickup chip 25A configured to acquire second image data according to the clock signal transmitted by one signal line from the first image pickup chip 22A, and a transmitting section 23A configured to transmit the first image data and the second image data by radio.

## Description

### Technical Field

Embodiments of the present invention relate to a capsule endoscope of a binocular type introduced into a body.

### Background Art

In recent years, a capsule endoscope including an image pickup function and a radio transmission function has emerged in the market. After being swallowed by an examinee, this capsule endoscope moves through insides of digestive tracts such as a stomach and a small intestine following a peristaltic movement and picks up images of insides of organs using the image pickup function until the capsule endoscope is naturally discharged.

Images picked up by an image pickup chip of the capsule endoscope while the capsule endoscope moves in the digestive tracts are transmitted to an external device provided on an outside of a subject as an image signal by the radio transmission function and stored in a memory of the external device. After swallowing the capsule endoscope, the examinee can freely act by carrying the external device including a radio reception function and a memory function. After observation by the capsule endoscope, the images stored in the memory of the external device are displayed on a display or the like and diagnosis or the like is performed.

A capsule endoscope of a so-called binocular type disclosed in Japanese Patent No. 4602828 includes image pickup chips respectively at both ends on a front side and a rear side of an elongated capsule type housing and picks up an image on the front side and an image on the rear side. In the capsule endoscope of the binocular type, in order to perform a stable operation in which the two image pickup chips are completely synchronized, it is preferable that the two image pickup chips share an oscillation signal generated by one transducer section.

Two signal lines are necessary for transmission of the oscillation signal. However, layout design of a wiring board housed in a small housing is not easy and is likely to cause an increase in a wiring board area, that is, an increase in a size of a housing.

It is an object of embodiments of the present invention to provide a small-diameter capsule endoscope that operates stably.

### Disclosure of Invention

### Means for Solving the Problem

According to an aspect of the present invention, there is provided a capsule endoscope in which, inside a housing of a capsule type, a circuit board formed by arranging a plurality of board sections to one another in a row via connecting sections is bent at 180 degrees in the plural connecting sections and the plurality of board sections are arranged such that principal planes of the respective board sections are orthogonal to a center axis of the housing, the capsule endoscope including: a transducer section for generating a clock signal; a first image pickup chip connected to the transducer section via two signal lines and configured to generate the clock signal and acquire first image data according to the generated clock signal; a second image pickup chip configured to acquire second image data according to the clock signal transmitted by one signal line from the first image pickup chip; and a transmitting section configured to transmit the first image data and the second image data by radio.

### Brief Description of the Drawings

Fig. 1 is an external view of a capsule endoscope in an embodiment.
Fig. 2 is a sectional view of a capsule endoscope in a first embodiment.
Fig. 3A is a top view for explaining a circuit board before mounting of the capsule endoscope in the first embodiment.
Fig. 3B is a bottom view for explaining the circuit board before mounting of the capsule endoscope in the first embodiment.
Fig. 3C is a sectional view taken along line IIIC-IIIC of Fig. 3A and Fig. 3B for explaining the circuit board before mounting of the capsule endoscope in the first embodiment.
Fig. 4A is a top view for explaining component mounting on the circuit board of the capsule endoscope in the first embodiment.
Fig. 4B is an exploded sectional view taken along line IVB-IVB of Fig. 4A for explaining the component mounting on the circuit board of the capsule endoscope in the first embodiment.
Fig. 5 is a configuration diagram of the capsule endoscope in the first embodiment.
Fig. 6A is a top view for explaining a circuit board of a capsule endoscope in a second embodiment.
Fig. 6B is a sectional view taken along line VIB-VIB of Fig. 6A for explaining the circuit board of the capsule endoscope in the second embodiment.

### Best Mode for Carrying Out the Invention

### <First Embodiment>

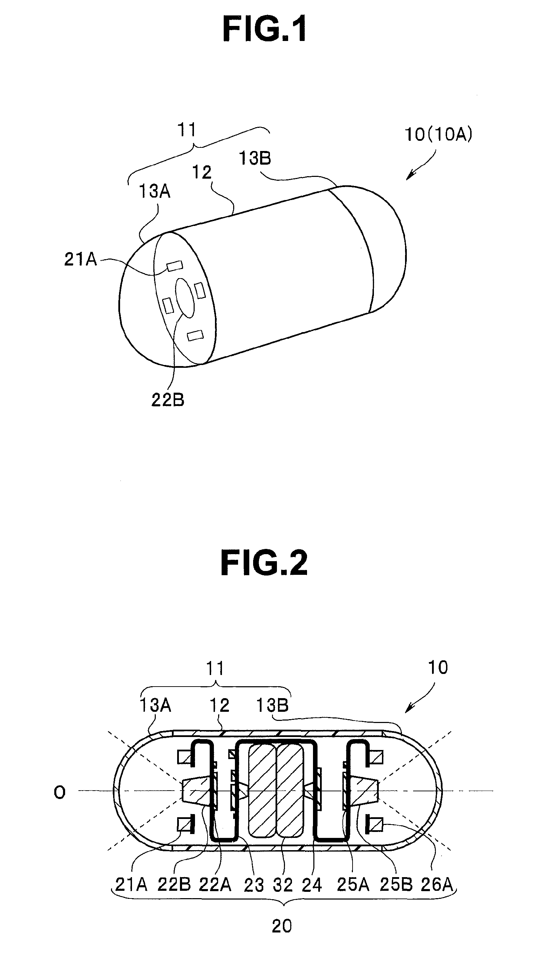

As shown in Fig. 1 and Fig. 2, in a capsule endoscope (hereinafter referred to as "endoscope") 10 in the present embodiment, an elongated circuit board 20 is housed inside a housing 11 of a capsule type in a bent state together with a battery 32.

The housing 11 includes a cylindrical main body section 12 and substantially semispherical end cover sections 13A and 13B at both ends of the main body section 12. The end cover sections 13A and 13B are made of a transparent material. The main body section 12 is made of an opaque material. The elongated housing 11 has a rotationally symmetrical shape having a center axis O in a longitudinal direction as an axis of rotational symmetry. Length L of the housing 11, that is, length L in a direction of the center axis O is 25 to 35 mm. A diameter D in an orthogonal direction of the center axis O is 5 to 15 mm.

Inside the housing 11 are housed a plurality of substantially circular board sections in a state in which respective connecting sections of a circuit board 20 having flexibility formed by arranging the plurality of substantially circular board sections in a row respectively via the connecting sections are bent at 180 degrees (90 degrees + 90 degrees) and principal planes of the respective board sections are orthogonal to the center axis O.

An image in a body in a front illuminated by light emitting elements 21 A arranged on the end cover section 13A side is acquired by a first image pickup chip 22A via a lens unit 22B. On the other hand, an image in the body in a rear illuminated by light emitting elements 26A arranged on the end cover section 13B side is acquired by a second image pickup chip 25A via a lens unit 25B. Note that, in the following explanation, for convenience, "front" refers to "end cover section 13A side" and "rear" refers to "end cover section 13B side".

The battery 32, which is a power supply source, is disposed between a power supply board section 24 and a transmission board section 23 of the circuit board 20 in a bent state.

Note that the circuit board 20 is housed in a housing together with a spacer member (not shown in the figure) for determining arrangement of the respective board sections.

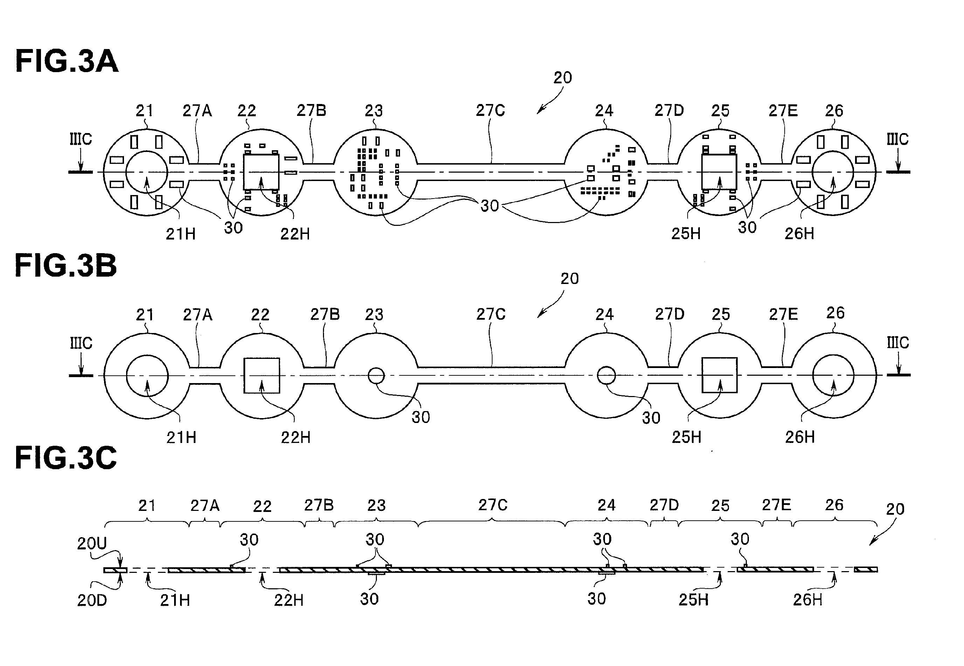

Next, the circuit board 20 is explained using Fig. 3A to Fig. 4B. Fig. 3A is a top view observed from a first principal plane 20U side, which is a mounting surface of the circuit board 20 before mounting of electronic components. Fig. 3B is a bottom view observed from a second principal plane 20D side of the circuit board 20 before mounting. Fig. 3C is a sectional view taken along line IIIC-IIIC of Fig. 3A and Fig. 3B.

The circuit board 20 is configured by arranging, in order, a first lighting board section 21, a connecting section 27A, a first image pickup board section 22, a connecting section 27B, the transmission board section 23, a connecting section 27C, the power supply board section 24, a connecting section 27D, a second image pickup board section 25, a connecting section 27E, and a second lighting board section 26 in a row. Note that, in the following explanation, each of the connecting sections 27A to 27E is referred to as connecting section 27.

On the circuit board 20, a plurality of connection electrodes 30 for mounting electronic components are formed. The connection electrodes 30 are made of a conductive material such as copper or gold. Mounting of the electronic components and the like on the circuit board 20 is performed by an SMT (surface mount technology) process. Openings 21H, 22H, 25H, and 26H are respectively provided in the board sections 21, 22, 25, and 26 of the circuit board 20.

Note that, although not shown in the figure, a plurality of wires for connecting the board sections are formed on the first principal plane 20U and the second principal plane 20D. For example, an L/S (line/space) of the wires is 75µm/75µm. One wire occupies width of 150 µm.

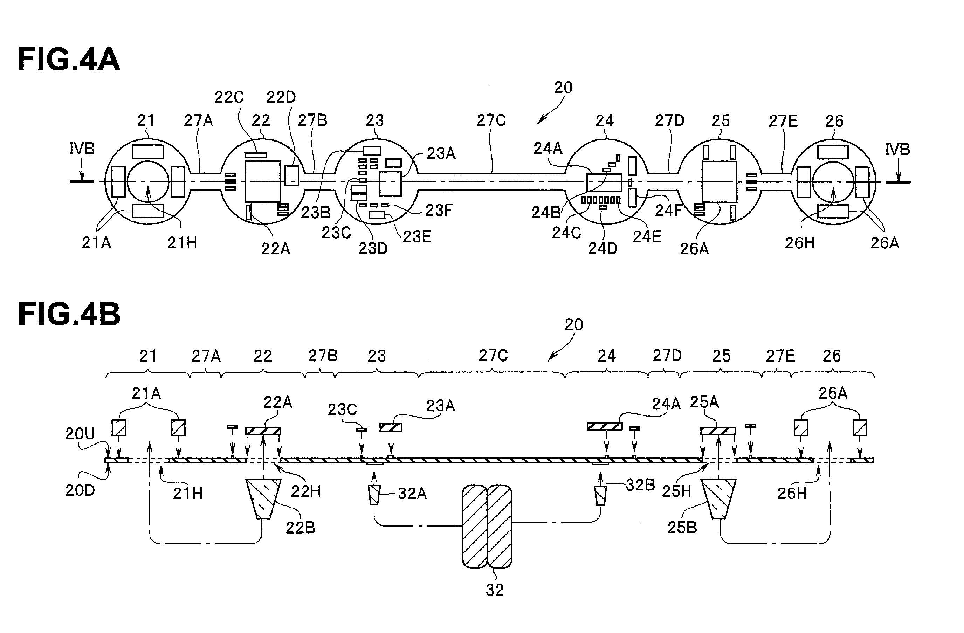

Next, Fig. 4A is a top view observed from the first principal plane 20U side of the circuit board 20 on which the electronic components and the like are mounted. Fig. 4B is an exploded sectional view taken along line IVB-IVB of Fig. 4A.

On the first principal plane 20U of the substantially circular first lighting board section 21, four light emitting elements 21A, for example, LEDs are mounted on the connection electrodes 30 around the substantially circular opening 21H. Note that the light emitting elements 21A are not limited to the LEDs. The number of the light emitting elements 21A is not limited to four.

The first image pickup chip 22A is flip-chip mounted on the first principal plane 20U of the substantially circular first image pickup board section 22 in a state in which an image pickup surface is directed to a side of the substantially rectangular opening 22H. In addition to a transducer section 22C, an EEPROM (22D), and the like, a lens unit 22B is arranged on the image pickup surface. As the image pickup chip 22A, a CCD, a CMOS image sensor, or the like is used.

A transmission IC, which is a transmitting section 23A configured to control radio transmission of an image signal, and other chip components (23B to 23F) are mounted on the first principal plane 20U of the substantially circular transmission board section 23. Although not shown in the figure, a coil pattern, which is an antenna for transmission, is formed in an inner layer of the transmission board section 23 having multiple wiring layers.

On the first principal plane 20U of the substantially circular power supply board section 24, in addition to a power supply IC, which is a power supply section 24A, chip components such as a resistor 24C, a capacitor 24D, a diode 24E, and an inductor 24F are surface-mounted. A convex contact member 32A for battery connection is formed on the other surface.

On the substantially circular image pickup board section 25, the second image pickup chip 25A is mounted. The lens unit 25B is arranged on an image pickup surface. Note that, unlike the first image pickup board section 22, a transducer section is not mounted on the second image pickup board section 25.

On the substantially circular second lighting board section 26, four light emitting elements 26A, for example, LEDs are mounted around the substantially circular opening 26H.

Note that various electronic components other than the electronic components and the like explained above are also mounted on the circuit board 20.

Wires, which are a plurality of signal lines, are formed in the connecting section 27 at high density.

The opening 21H in the center of the first lighting board section 21 located at one end of the circuit board 20, which is an integral long flexible board, is bent to cover a frame of the lens unit 22B. The opening 26H in the center of the second lighting board section 26 located at the other end is bent to cover a frame of the lens unit 25B. Therefore, an assembly process for the circuit board 20 is possible by bending the circuit board 20 in order along the longitudinal direction and is easy because complicated bending work is unnecessary.

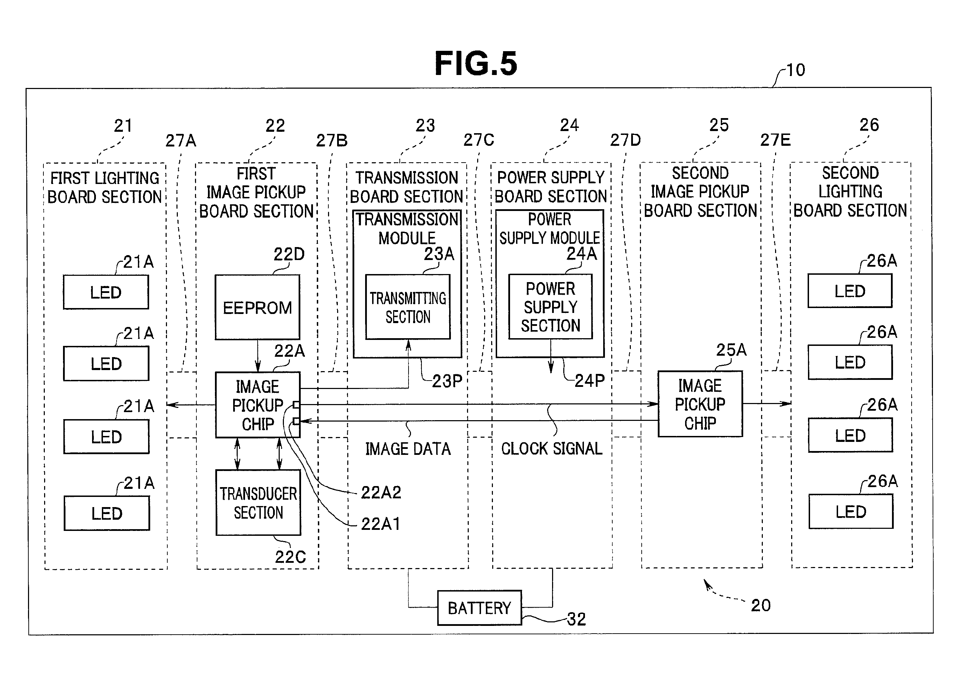

Next, a configuration of the endoscope 10 is further explained using Fig. 5.

The transducer section 22C mounted on the first image pickup board section includes a crystal transducer configured to generate an oscillation signal having a predetermined natural frequency in an oscillation circuit combined with a transistor and a capacitor. The oscillation signal generated by the transducer section 22C is transmitted by short two signal lines to the first image pickup chip 22A arranged near the transducer section 22C.

The first image pickup chip 22A reduces a frequency of the inputted oscillation signal with a frequency dividing circuit therein and generates a clock signal used for control. The first image pickup chip 22A controls light emission timing and image pickup timing of the light emitting elements 21A of the first lighting board section 21 on the basis of the generated clock signal. The EEPROM (22D) reads and writes operation parameters of the image pickup chip.

The first image pickup chip 22A transmits acquired first image data to the transmitting section 23A of the transmission board section 23 arranged adjacent to the first image pickup chip 22A via the connecting section 27 together with the clock signal. That is, since the image data needs to be transmitted together with the clock signal, two signal lines are necessary for transmission of the image data.

The transmitting section 23A transmits the inputted first image data by radio.

On the other hand, the second image pickup chip 25A controls light emission timing and image pickup timing of the light emitting elements 26A of the second lighting board section 26 on the basis of the clock signal transmitted from the first image pickup chip 22A by one signal line.

As explained above, in order to perform a stable operation in which the second image pickup chip 25A is completely synchronized with the first image pickup chip 22A, the second image pickup chip 25A needs to share an oscillation signal generated by the same transducer section 22C. However, the transducer section 22C needs long two signal lines to directly transmit the generated oscillation signal to the second image pickup chip 25A. On the other hand, since the clock signal is a pulse voltage signal with respect to a ground potential, the clock signal can be transmitted by one signal line inside the circuit board 20 that shares an earth wire.

The first image pickup chip 22A includes an electrode terminal for clock signal transmission 22A1 for transmitting the clock signal generated on the basis of the oscillation signal to an outside. The second image pickup chip 25A controls image pickup timing and the like on the basis of the clock signal transmitted from the first image pickup chip 22A. Therefore, when the first image pickup chip 22A is a "master", the second image pickup chip 25A is a "salve". The "master" and the "slave" do not operate independently from each other. A so-called master/slave operation is performed in which the "master" controls driving timing of the "slave".

In the endoscope 10, the first image pickup chip 22A transmits the clock signal to the second image pickup chip 25A. Therefore, one signal line from the first image pickup board section 22 to the second image pickup board section 25 can be reduced. That is, wires passing the connecting section 27B, the transmission board section 23, the connecting section 27C, the power supply board section 24, and the connecting section 27D may be less by one.

Further, the first image pickup chip 22A includes an electrode terminal for image data input 22A2. Second image data acquired by the second image pickup chip 25A can be inputted to the first image pickup chip 22A. That is, the first image pickup chip 22A has a function of outputting not only the first image data picked up by the first image pickup chip 22A but also the second image data inputted from the second image pickup chip 25A. Therefore, the second image data is also transmitted to the transmitting section 23A via the first image pickup chip 22A.

Only one image data can be simultaneously inputted to the transmitting section 23A. Therefore, in order to input the first image data and the second image data to the transmitting section 23A, it is necessary to adjust timing for the first image pickup chip 22A to output the first image data and timing for the second image pickup chip 25A to output the second image data.

However, in the endoscope 10, since the second image data is transmitted to the transmitting section 23A via the first image pickup chip 22A, the timing adjustment is easy. Further, to transmit the second image data to the transmitting section 23A, the one signal line to the first image pickup chip 22A is used instead of the two signal lines from the second image pickup chip 25A to the transmitting section 23A.

In order to connect the signal line to the two image pickup chips 22A and 25A arranged in separated positions in the circuit board 20, it is necessary to perform wiring layout design for the signal line to avoid a component mounting pattern and the like. Therefore, when the signal line is disposed, a necessary diameter of a circular board section present in a wiring route needs to be increased by a wire disposing space. For example, when two wires are additionally disposed, a diameter of the circular board section increases by 0.3 mm. This is a significant influence for the circuit board 20 including the circular board section having a diameter of several millimeters.

In the endoscope 10, an area of the circuit board 20 can be reduced because the transmitting section 23A and the second image pickup chip 25A, which is the "slave", are not connected by a signal line. Therefore, it is possible to design the endoscope to be small in diameter. The first image pickup chip 22A, which is the "master", and the transmitting section 23A are connected at a short distance via the connecting section 27B. Therefore, noise is less easily superimposed on an image signal transmitted by radio.

The transducer section 22C for generating a clock signal for specifying operation timings of the image pickup chips 22A and 25A is mounted in a position adjacent to the first image pickup chip 22A of the first image pickup board section 22 and electrically connected to the first image pickup chip 22A.

When the transducer section 22C and the two image pickup chips 22A and 25A are respectively connected by signal lines, as in the case of the connection to the transmitting section 23A, a necessary diameter of a circular board section present in a wiring route has to be increased. On the other hand, in the endoscope 10, the transducer section 22C is connected via the two signal lines to the first image pickup chip 22A arranged in the same first image pickup board section 22 but the second image pickup chip 22A is not connected to the transducer section 22C. However, the first image pickup chip 22A and the second image pickup chip 22A are controlled by the same clock signal obtained by reducing a frequency of an oscillation signal generated by one transducer section 22C.

Therefore, the endoscope 10 operates stably and can be designed to be small in diameter because the number of wires in the circuit board 20 is small.

### <Second Embodiment>

Next, an endoscope 10A in a second embodiment is explained. Since the endoscope 10A is similar to the endoscope 10, the same components are denoted by the same reference numerals and signs and explanation of the components is omitted.

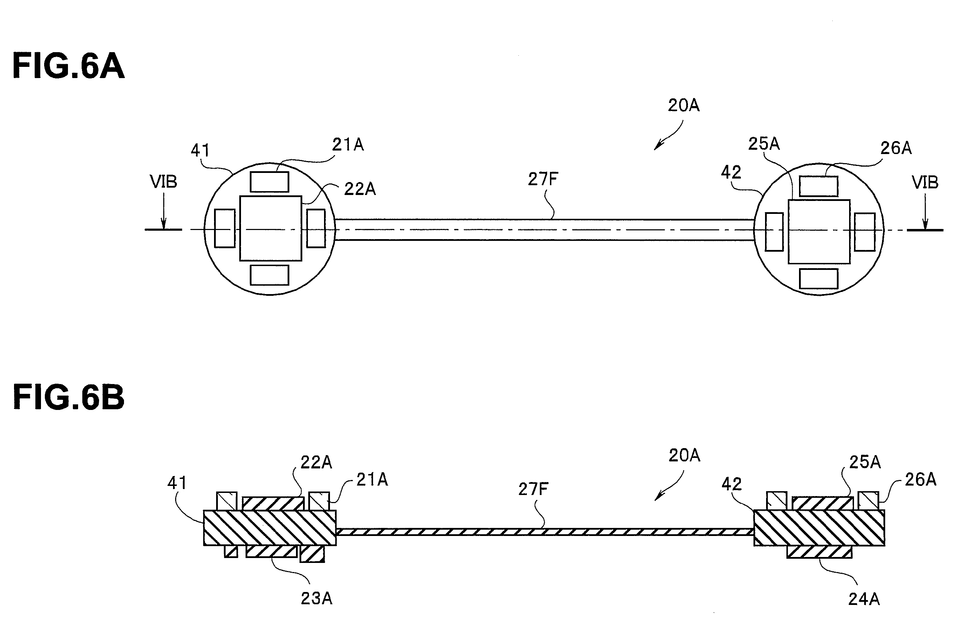

As shown in Fig. 6A and Fig. 6B, a circuit board 20A of the endoscope 10A is a so-called rigid flexible wiring board in which board sections 41 and 42, which are two substantially circular multilayer wiring boards, are connected via a connecting section 27F. The board section 41 is a master board section mounted face up such that the first image pickup chip 22A faces an opposite direction of the board section. The board section 41 is a multilayer wiring board including all the functions of the first lighting board section 21, the connecting section 27A, the first image pickup board section 22, the connecting section 27B, and the transmission board section 23 of the circuit board 20. On the other hand, the board section 42, which is a slave board section, is a multilayer wiring board including all the functions of the power supply board section 24, the connecting section 27D, the second image pickup board section 25, the connecting section 27E, and the second lighting board section 26.

An antenna of the transmitting section 23A is formed by an inner layer pattern of the board section 41, which is the multilayer wiring board. Peripheral components of the transmitting section 23A are also mounted on a rear surface of the board section 41. That is, the transmitting section 23A is arranged in a state in which the transmitting section 23A is adjacent to the first image pickup chip 22A via a distance equivalent to thickness of the board section 41. The transmitting section 23A is not directly connected to the second image pickup chip 25A.

With such a configuration, in the endoscope 10A, wires for connecting the transmitting section 23A and the first image pickup chip 22A are further simplified. Further, since a wire for transmitting image data is shorter in the circuit board 20A than in the circuit board 20, noise is further reduced.

Note that the transducer section 22C and the EEPROM (22D) are mounted on the rear surface of the board section 41 and adjacent and electrically connected to the first image pickup board section 22.

The second image pickup chip 25A is mounted face up on the board section 42. The light emitting elements 26A are mounted around the second image pickup chip 25A. The power supply section 24A is mounted on a rear surface of the board section 42.

In the endoscope 10A, the configuration of the circuit board 20A is simplified compared with the circuit board 20 of the endoscope 10.

Therefore, the endoscope 10A has the same effect as the endoscope 10. An assembly process for the endoscope 10A can be further simplified.

The present invention is not limited to the embodiments explained above and various alterations, modifications, and the like are possible in a range in which the gist of the present invention is not changed.

This application is filed claiming priority from Japanese Patent Application No. 2011-189701 filed in Japan on August 31, 2011, disclosed contents of which are cited in the specification of this application, the claims, and the drawings.

## Claims

1. A capsule endoscope in which, inside a housing of a capsule type, a circuit board formed by arranging a plurality of board sections to one another in a row via connecting sections is bent at 180 degrees in the plural connecting sections and the plurality of board sections are arranged such that principal planes of the respective board sections are orthogonal to a center axis of the housing, the capsule endoscope comprising:
a transducer section for generating a clock signal;
a first image pickup chip connected to the transducer section via two signal lines and configured to generate the clock signal and acquire first image data according to the generated clock signal;
a second image pickup chip configured to acquire second image data according to the clock signal transmitted by one signal line from the first image pickup chip; and
a transmitting section configured to transmit the first image data and the second image data by radio.

2. The capsule endoscope according to claim 1, wherein the second image data is transmitted to the transmitting section via the first image pickup chip.

3. The capsule endoscope according to claim 2, further comprising:
a first lighting board section on which a plurality of light emitting elements configured to illuminate an image pickup visual field of the first image pickup chip are mounted;
a first image pickup board section on which the transducer section and the first image pickup chip are mounted;
a transmission board section on which the transmitting section is mounted;
a power supply board section on which a power supply section configured to supply electric power is disposed;
a second image pickup board section on which the second image pickup chip is mounted; and
a second lighting board section on which a plurality of light emitting elements configured to illuminate an image pickup visual field of the second image pickup chip are mounted.

4. The capsule endoscope according to claim 3, wherein the transmission board section is arranged adjacent to the first image pickup board section via the connecting section.

5. The capsule endoscope according to claim 3, wherein the circuit board includes a first multilayer board section formed by integrating the first image pickup board section, the first lighting board section, and the transmission board section 23, a second multilayer board section formed by integrating the second image pickup board section, the second lighting board section, and the power supply board section, and a connecting section for connecting the first multilayer board section and the second multilayer board section.
